# EUROPEAN PATENT SPECIFICATION

(11) **EP 1 651 145 B1**
(45) Date of publication and mention of the grant of the patent: **16.11.2016**
(21) Application number: 04757367.0
(22) Date of filing: 28.07.2004
(51) Int. Cl.: A61F 2/16

(54) **PRIMARY AND SUPPLEMENTAL INTRAOCULAR LENS SYSTEM**
PRIMÄRES UND ERGÄNZENDES INTRAOKULARLINSEN-SYSTEM
SYSTEME DE LENTILLE INTRAOCULAIRE PRIMAIRE ET COMPLEMENTAIRE

(30) Priority: 28.07.2003 US 629210
(43) Date of publication of application: 03.05.2006
(73) Proprietor: Abbott Medical Optics Inc., Santa Ana, CA 92705-4933 (US)
(72) Inventor: BRADY, Daniel, G., San Juan Capistrano, CA 92675 (US); ROCKLEY, Paul, Corona del Mar, CA 92625 (US)
(74) Representative: Hoffmann Eitle
(86) International application number: PCT/US2004/024399
(87) International publication number: WO 2005/011531

(56) References cited:
- EP-A- 0 537 643
- WO-A-02/060346
- WO-A-03/000154
- US-A- 5 824 074
- US-B1- 6 197 057
- US-B1- 6 596 026

## Description

### Field of the Invention

This invention relates generally to intraocular lenses and, more particularly, to supplemental intraocular lenses, which can be placed in, on, or near primary intraocular lenses to change the effective optical power of the primary intraocular lens.

### Background of the Invention

Vision is achieved in the human eye by transmitting an image through a clear outer portion called the cornea, and focusing this image via a natural lens onto a retina. When the natural lens loses its ability to clearly focus the image onto the retina through, for example, cataracts or injury, the quality of the focused image on the retina can be severely compromised.

An accepted treatment for a damaged natural lens is surgical removal of the natural lens and replacement of the natural lens with an artificial intraocular lens. One way to accomplish this procedure is to form a relatively long incision in the eye and remove the natural lens in one piece. A more popular method for removing the natural lens is to form a shorter incision in the eye and insert a probe or a phaco tip of a phacoemulsification instrument through the incision into the eye to break up the natural lens using ultrasonic energy. The lens fragments can then be aspirated from the natural eye through the relatively short phaco incision, and the phaco tip is then removed.

A preferred conventional method of removing a natural lens is accompanied with a subsequent implantation of a replacement intraocular lens in the same surgical procedure. A typical intraocular lens includes an optic usually having a diameter of about 6mm, and fixation members coupled to (or formed with) the optic to fix the optic within the eye in the region of the extracted natural lens. These fixation members are generally in the form of at least two haptics, which may be flexible, elongated, openended loops that project from the edge of an optic portion of the intraocular lens. The fixation member may require additional incision links, depending upon the number, length, and configuration of the fixation member.

Intraocular lenses can be of two basic types, those having a hard or rigid optic formed, for example, of polymethyl methacrylate (PMMA) and those having a deformable optic which is constructed of a deformable material such as silicone, hydrogel, or an acrylic. When a hard intraocular lens is used, the small phaco incision must be enlarged to approximately the diameter of the hard optic, in order to permit the hard optic to be inserted through the incision. A deformable optic, on the other hand, may have a high elongation so that the optic can be resiliently stretched and flexed to assume a small cross-sectional configuration for passage through a small phaco incision.

Before implanting the intraocular lens, the physician must determine the intraocular lens power needed to achieve the desired refraction needs of the patient. This procedure can be difficult and inexact. Errors in measurement, inaccuracy of assumptions, and the difficulty of achieving precise placement of an intraocular lens make the physician's selection of an exact corrective power highly prone to inaccuracies. Post-operative changes to the patient's eye may also change the refractive power needed for the intraocular lenses in the patient. Consequently, the intraocular lens, after implantation, does not always provide a perfect vision correction. These post-operative refractive errors must often be corrected by a subsequent surgery to replace the implanted intraocular lens with another intraocular lens. A subsequent surgery involves re-entry into the eye through a new incision, removal of the initial intraocular lens, and implantation of a new intraocular lens. Needless to say, this conventional subsequent surgery procedure can be traumatic to the eye.

One approach for limiting the amount of trauma on the human eye caused by subsequent replacement of the intraocular lens is disclosed in Patel U.S. Patent 5,366,502. This patent discloses supplemental intraocular lenses which may be subsequently attached to primary intraocular lenses after the initial implantation of the primary intraocular lens. Addition of a supplemental intraocular lens to a primary intraocular lens does not entail removal of the primary intraocular lens, and further requires a relatively small incision in the eye. The supplemental intraocular lenses, and most of the primary intraocular lenses, of this patent include specially configured connectors for mating the supplemental intraocular lens to the implanted, primary intraocular lens. These connectors can be in the form of hooks, projections, slots, and loops, which are suitable for securing the supplemental intraocular lens to the primary intraocular lens. These various securing means, however, can be complex and difficult to manufacture and implement. Additionally, the sizes of these supplemental intraocular lenses are often unnecessarily large, thus requiring a larger incision and more trauma to the eye.

One attempt to overcome some of the aforementioned problems is disclosed in Portney U.S. Pat. No. 6,454,801, which discusses various alternative arrangements for securing the supplemental intraocular lens to the primary intraocular lens. In one embodiment, the supplemental intraocular lens is provided with a semi-rigid annular lip that wraps around and clamps against the primary intraocular lens. In another embodiment, the supplemental intraocular lens is secured to the primary intraocular lens with a biological glue or other suitable adhesive. In still another embodiment, the primary intraocular lens is provided with a pocket for receiving the supplemental intraocular lens.

US 6,596,026 discloses an intraocular lens implant including a telescope body defining an optical path for light to pass therethrough, a positive lens and a negative lens attached to the telescope body, and mounting structure connected to the telescope body for mounting the implant in an eye, wherein the lenses abut against each other, and a distance between the positive lens and the negative lens is fixed and determined by the lenses abutting against each other.

One concern associated with supplemental intraocular lens systems is the potential for cellular deposits to form between the two lenses. Such deposits could result in opacification of the optics and impairment of vision.

Another potential concern is that conventional refractive lenses must be made relatively thick to avoid distortion when the lenses are subjected to external forces. For instance, low diopter refractive lenses typically have a minimum center thickness of at least about 700 /an, while higher diopter refractive lenses are even thicker. Thus, the combined thicknesses of a primary intraocular lens and a supplemental refractive intraocular lens may be too much for the confined space within the anterior or posterior chambers of the eye. Furthermore, thick supplemental lenses require relatively long surgical incisions. It is generally desirable to keep the incisions as short as possible in order to avoid surgical trauma and decrease the patient's recovery time.

Accordingly, it would be advantageous to provide new and improved primary and supplemental intraocular lens systems wherein the combined thickness of, and the potential for cellular growth between, the two lenses is reduced, and wherein optical distortions from external forces on the lenses are reduced.

### SUMMARY OF THE INVENTION

The invention is defined by the subject matter of independent claim 1, wherein the dependent claims describe further aspects of the invention.

In accordance with the present invention, new primary and supplemental intraocular lens systems have been discovered. Such systems comprise a primary intraocular lens configured for placement in an eye of a patient and to be effective in correcting vision of the patient, and a supplemental intraocular lens configured for placement in the eye of the patient and to modify the correction provided by the primary intraocular lens, wherein the supplemental intraocular lens is a substantially completely diffractive optic.

Because the supplemental intraocular lens is substantially completely diffractive, its refractive power is substantially independent of both the thickness of the optic and the refractive index of the material from which the optic is made. As a result, the supplemental intraocular lens can be made in the form of an extremely thin, or Aultrathin, membrane.

In one useful embodiment the supplemental intraocular lens has a thickness, for example a center thickness, of less than about 700µm, and is advantageously a meniscus-type lens. Preferably the thickness of the supplemental intraocular lens is in the range of about 10µm to about 300µm, and more preferably, the thickness of the supplemental intraocular lens is no more than about 250µm.

The supplemental intraocular lens may be either connected to, or separate from, the primary intraocular lens. In one advantageous embodiment, the supplemental intraocular lens is connected to and anteriorly vaulted with respect to the primary intraocular lens. The anterior vaulting of the supplemental intraocular lens allows for sufficient spacing between the two lenses to inhibit the formation of cellular deposits.

The supplemental intraocular lens may have multifocal correction, cylindrical correction, wavefront correction, and/or spherical correction to augment the primary intraocular lens. The supplemental intraocular lens may also include a blue blocker and/or other color/UV filter material, in accordance with a patients specific needs. The primary intraocular lens may be a conventional refractive lens, or may be a thin diffractive lens substantially similar to the supplemental intraocular lens.

Each and every feature described herein, and each and every combination of two or more of such features, is included within the scope of the present invention provided that the features included in such a combination are not mutually inconsistent.

Additional aspects, features, and advantages of the present invention are set forth in the following description and claims, particularly when considered in conjunction with the accompanying drawings in which like parts bear like reference numbers.

### Brief Description of the Drawings

Fig. 1 is a vertical cross-section of an eye illustrating an exemplary primary/supplemental intraocular lens system of the present invention positioned within the capsular bag;
Fig. 2 is a view similar to Fig. 1, showing an alternate embodiment of the invention wherein the primary intraocular lens is positioned within the capsular bag and the supplemental intraocular lens is located in the sulcus;
Fig. 3 is an enlarged vertical cross-section of the primary/supplemental intraocular lens system of Fig. 1;
Fig. 4 is a top planar view of the primary/supplemental intraocular lens system of Fig. 3;
Fig. 5 is a cross-sectional view of a supplemental lens according to the present invention; and
Fig. 6 is a graphical illustration of an exemplary phase profile for the supplemental lens of Fig. 5.

### Description of the Preferred Embodiments

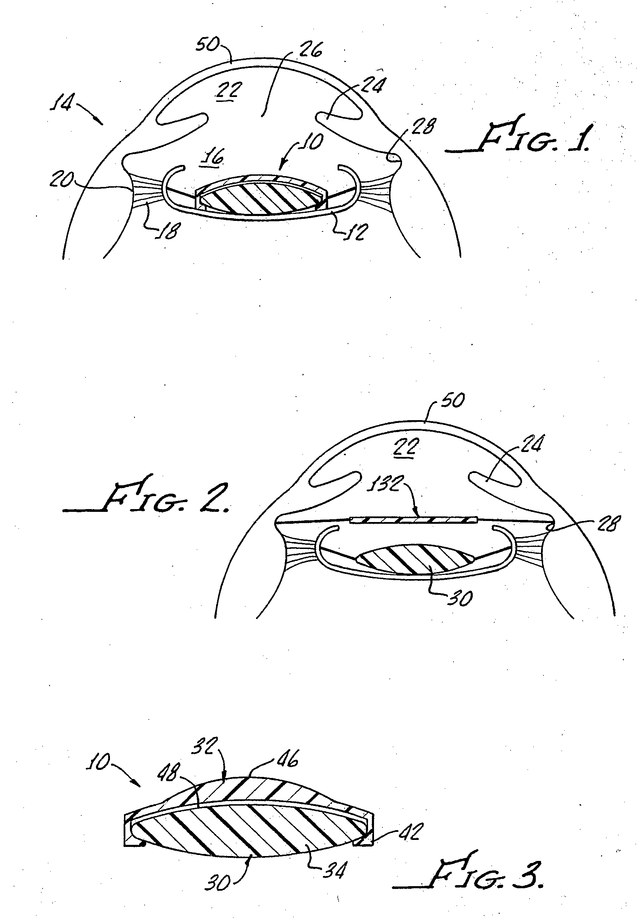

Referring to the drawings in more detail, Fig. 1 shows an intraocular lens system 10 according to the present invention implanted in the capsular bag 12 of an eye 14. The capsular bag 12 is held in the posterior chamber 16 of the eye 14 by a set of suspensory ligaments or zonules 18 that extend between the capsular bag 12 and an annular ciliary muscle 20. The posterior chamber 16 is separated from the anterior chamber 22 of the eye 14 by an annular iris 24, which defines the variable opening or aperture known as the pupil 26. The iris is separated from the ciliary muscle by an annular groove known as the sulcus 28.

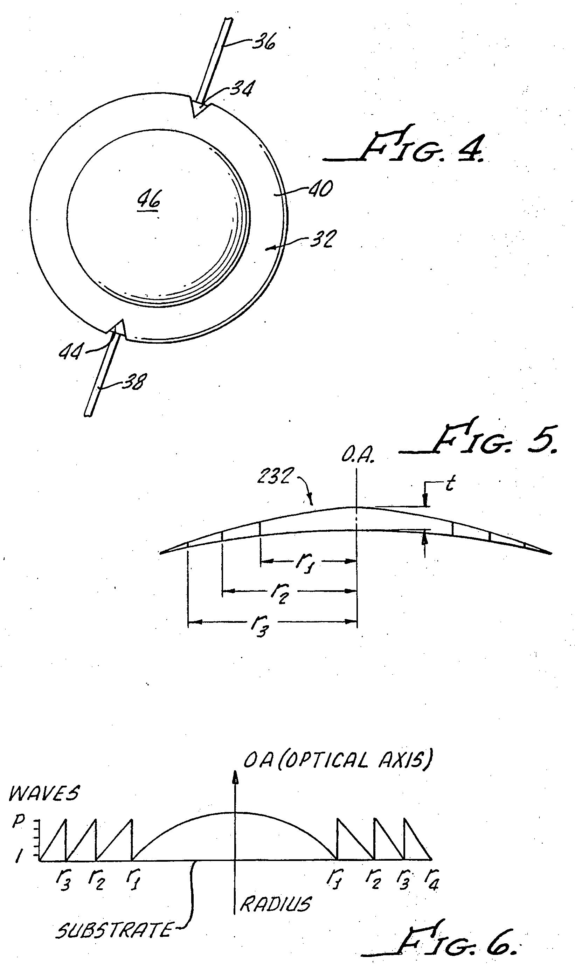

Turning now to Figs. 3 and 4, the intraocular lens system 10 includes a primary intraocular lens 30 that is configured to correct the vision of a patient, and a supplemental intraocular lens 32 that is configured to modify the correction of the primary intraocular lens 30. The supplemental intraocular lens 32 may be implanted simultaneously with the primary intraocular lens 30, or added in a subsequent surgical procedure, shortly thereafter or years iater.

The primary intraocular lens 30 includes an optic body 34 and fixation members or haptics 36, 38 for positioning the optic body 34 in the capsular bag 12. The optic body 34 need not be limited to the biconvex refractive configuration shown here, but may also have a plano-convex or concave-convex refractive configuration, a diffractive or refractive/diffractive hybrid configuration, or the like. Similarly, the fixation members 36, 38 need not be limited to the filament-type haptics shown here, but may have any suitable configuration.

The supplemental intraocular lens 32, which is not drawn to scale, but has its thickness exaggerated for purposes of illustration, is a diffractive lens, for instance a multi-order diffractive (MOD) lens of the type shown in Faklis et al. U.S. Patent No. 5,589,982. The disclosure of this U.S. patent is incorporated in its entirety herein by reference. Further information on the characteristics and design of multi-order diffractive (MOD) lenses is available in D. Faklis and G. M. Morris, ASpectral Properties of Multiorder Diffractive lenses, Applied Optics, Vol. 34, No. 14,2462-2468, the contents of which are also incorporated in their entirety herein by reference.

Substantially completely diffractive lenses of the type disclosed in the aforementioned Faklis et al. patent are sometimes known as Fresnel Zone Plates (FZPs). It is important to distinguish between Fresnel Zone Plates and Fresnel lenses, which have no diffractive power. Both Fresnel Zone Plates and Fresnel lenses have faceted zones. However, in Fresnel lenses the phase differences between zones are random, while in Fresnel Zone Plates, the phase differences are carefully controlled so that the light transmitted through each facet, or echelette, is coherently superposed with the light transmitted through the other facets/echelettes. In Fresnel lenses, any amplitude addition across the lens is insignificant, and no useable diffractive power is generated. In Fresnel Zone Plates, on the other hand, the amplitudes of the diffracted wavefronts combine to form a single new wavefront that is continuous across the entire aperture of the lens, resulting in the possibility of diffraction-limited performance. In summary, the power of a Fresnel lens is determined solely by refraction at each of the facets/echelettes, of the lens, while the power of a Fresnel Zone plate is determined by the diffractive effects, with the effects of refraction being secondary at best.

It is also important to distinguish between Fresnel Zone Plates, or diffractive lenses, of the type described above, and refractive/diffractive hybrid lenses. A typical refractive/diffractive hybrid lens has a diffractive profile formed on one of its two surfaces. The diffractive power of this surface is additional to the refractive power of the lens, which is a function of the curvature of the other surface, as well as of the material and thickness of the lens. Refractive/diffractive hybrid lenses, which are typically used to provide bifocal or multifocal vision correction, are not Asubstantially completely diffractive, as defined herein.

The substantially completely diffractive supplemental intraocular lens 32 may have one or more of a wide variety of optical characteristics, depending on the characteristics of the primary intraocular lens 30, as well as on the needs of the patient. For instance, the supplemental intraocular lens 32 may be either positively powered, if the add power of the primary intraocular lens 30 is insufficient, or negatively powered, if the add power of the primary intraocular lens 30 is excessive. Alternatively, or in addition, the supplemental intraocular lens 32 may add multifocal, toric, wavefront, or spherical correction to the primary intraocular lens, and may also include a UV filter or a tint, for instance a blue-blocker, for blocking out portions of the visible spectrum.

The supplemental intraocular lens 32 shown in Figs. 3 and 4 includes a stretchable peripheral zone 40 and a semi-rigid annular lip 42 that wraps around and clamps against the primary intraocular lens 30. Apertures 44 are provided for accommodating the fixation members 36, 38 of the primary intraocular lens 30.

Details of the illustrated attachment arrangement between the supplemental intraocular lens 32 and the primary intraocular lens 30 are disclosed in Portney U.S.

Patent No. 6,454,801, the disclosure of which is incorporated in its entirety herein by reference. However, the primary and supplemental intraocular lenses 30, 32 may also be attached by other means such as, for instance, biological glue, a pocket formed in the primary intraocular lens 30, or any of the arrangements disclosed in Patel U.S. Patent 5,366,502, the contents of which are also disclosed in their entirety herein by reference.

Preferably, the attachment arrangement selected should secure the edge or periphery of the supplemental intraocular lens 32 to the edge or periphery of the primary intraocular lens 30, while allowing the central portion 46 of the supplemental intraocular lens 32 to vault anteriorly of the primary intraocular lens 30. The anterior vaulting of the supplemental intraocular lens 32 creates a space 48 between the two intraocular lenses 30, 32, thereby reducing the potential for cellular growth therebetween.

An alternate embodiment of the invention is shown in Fig. 2, wherein a supplemental intraocular lens 132 is separate from the primary intraocular lens 30, and mounted in the ciliary sulcus 28. This arrangement may allow the supplemental intraocular lens 132 to be implanted more easily than the arrangement of Fig.1, and may encourage patients to undergo new lens implantations or explantations years after the original surgery, to take advantage of new technology as it becomes available, and to keep up with age-related changes in the patient=s vision.

Other potential locations for both the primary intraocular lens 30 and the supplemental intraocular lens 132 will be readily apparent, and are included within the scope of the invention. For instance, one or both lenses may be implanted on the iris 24, in the cornea 50, or in the anterior chamber 22. Also, more than one supplemental intraocular lens can be used with the primary intraocular lens 30, with each additional supplemental lens adding a new feature or improvement to the previously implanted system.

Fig. 5 illustrates an exemplary multi-order diffractive (MOD)lens 232 that may be used as a supplemental intraocular lens in either of the primary/supplemental intraocular lens combinations shown in Figs. 1 and 2. The diffractive lens 232 is an ultrathin concave-convex, or meniscus-type, lens formed of a pliable, optically transmissive material such as a silicone polymeric material, an acrylic polymeric material, a hydrogel material, or combination thereof. The diffractive lens 232 preferably has a maximum thickness t of less than about 700µm, regardless of the lens material=s index of refraction. Preferably, the thickness t is in the range of about 10µm to about 300µm, and more preferably, the thickness t is no more than about 250µm. A diffractive lens 232 having a thickness in this range will remain substantially free of optical distortions when subjected to external forces, in contrast to a refractive lens of the same thickness, which would be significantly more vulnerable to optical distortion.

The diffractive lens 232 is centered on an optical axis O.A., and includes a number of concentric, full period zones, with the zone boundaries located at radii r₁, r₂, r₃, and r₄. Each zone comprises a repetitive sequence of facets, or echelettes, each of the facets having a predetermined profile and depth. Typically, the depth of each echelette is on the order of a wavelength (□). Thus, the echelettes can not be seen by the naked eye, and are not illustrated herein.

Each zone is a full period Fresnel zone. The zones are configured so that light incident on the lens experiences an optical phase shift, and the zone boundaries diffract light of different wavelengths in a different diffractive order to a single focal point, thereby providing a plural or multiple order singlet.

Fig. 6 is a diagram showing an exemplary phase profile for the supplemental lens 232 of Fig. 5. The number of waves for each zone boundary is indicated as p and the phase jump of phases at each zone boundary is constant. This profile, known as a blaze profile, is described in detail in Faklis et al. U.S. Patent No. 5,589,982, the disclosure of which is incorporated in its entirety herein by reference. Other phase profiles, such as a phase reversal (or Wood) profile or a multi-order approximation to the blaze profile can also be used. First-order diffractive profiles may be acceptable as well, but offer the designer less freedom.

While the present invention has been described with respect to various specific examples and embodiments, it is to be understood that the invention is not limited thereto and that it can be variously practiced within the scope of the following claims.

## Claims

1. An intraocular lens system for insertion into an eye, comprising:
a primary intraocular lens (30) configured for placement in an eye of a patient; and
a supplemental intraocular lens (32; 132; 232) configured for placement in the eye of the patient, the supplemental intraocular lens (32; 132; 232) comprising a substantially completely diffractive optic, **characterized in that**
the primary intraocular lens (30) is configured to be effective in correcting vision of the patient and the supplemental intraocular lens (32; 132; 232) is configured to modify the vision correction provided by the primary intraocular lens (30), and the supplemental intraocular lens (32; 132; 232) is either anteriorly vaulted with respect to the primary intraocular lens (30) or toric.

2. An intraocular lens system according to claim 1, wherein the supplemental intraocular lens (32; 132; 232) is configured to enhance the vision correction provided by the primary intraocular lens (30).

3. The intraocular lens system according to any one of the preceding claims, wherein the supplemental intraocular lens (32; 132; 232) comprises a resiliently bendable lens.

4. The intraocular lens system according to any one of the preceding claims, wherein the supplemental intraocular lens (32; 132; 232) has a thickness of less than about 700 µm.

5. The intraocular lens system according to any one of the preceding claims, wherein the supplemental intraocular lens (32; 132; 232) has a thickness in the range of about 10 µm to about 300 µm.

6. The intraocular lens system according to claim 5, wherein the supplemental intraocular lens (32; 132; 232) has a thickness of no more than about 250 µm.

7. The intraocular lens system according to any one of the preceding claims, wherein the supplemental intraocular lens (32; 132; 232) is operatively coupled to the primary intraocular lens (30).

8. The intraocular lens system according to any one of the preceding claims, wherein the supplemental intraocular lens (32; 132; 232) has a positive optical power.

9. The intraocular lens system according to any one of the preceding claims, wherein the supplemental intraocular lens (32; 132; 232) has a negative optical power.

10. The intraocular lens system according to any one of the preceding claims, wherein the supplemental intraocular lens (32; 132; 232) is tinted.

11. The intraocular lens system according to claim 10, wherein the supplemental intraocular lens (32; 132; 232) includes a blue blocker.

12. The intraocular lens system according to any one of the preceding claims, wherein the supplemental intraocular lens (32; 132; 232) is multifocal.

13. The intraocular lens system according to any one of the preceding claims, wherein the supplemental intraocular lens (32; 132; 232) has a refractive power and a thickness, the refractive power being independent of the thickness.

14. The intraocular lens system according to any one of the preceding claims, wherein the supplemental intraocular lens (32; 132; 232) has a refractive power and is formed of a material having an index of refraction, the refractive power of the supplemental intraocular lens (32; 132; 232) being independent of the index of refraction of the material.

15. The introocular lens system according to any one of the preceding claims, wherein the primary intraocular lens (30) has fixation members (36, 38) for fixing the primary intraocular lens (30) in an eye of a patient, and
the supplemental intraocular lens (32; 132; 232) is configured for attachment to the primary intraocular lens (30) or implantation in the eye of the patient separate from the primary intraocular lens (30).

## Patentansprüche

1. Intraokularlinsensystem zum Einführen in ein Auge, umfassend:
eine primäre Intraokularlinse (30), gestaltet zum Anordnen in einem Auge eines Patienten; und
eine Ergänzungs-Intraokularlinse (32; 132; 232), gestaltet zum Anordnen in einem Auge des Patienten, wobei die Ergänzungs-Intraokularlinse (32; 132; 232) eine im Wesentlichen vollständig beugende Optik umfasst, **dadurch gekennzeichnet, dass**
die primäre Intraokularlinse (30) dafür gestaltet ist, zum Korrigieren des Sehens des Patienten wirksam zu sein, und die Ergänzungs-Intraokularlinse (32; 132; 232) dafür gestaltet ist, die von der primären Intraokularlinse (30) geleistete Sehkorrektur zu modifizieren, und die Ergänzungs-Intraokularlinse (32; 132; 232) entweder anterior gewölbt bezogen auf die primäre Intraokularlinse (30) oder torisch ist.

2. Intraokularlinsensystem gemäß Anspruch 1, wobei die Ergänzungs-Intraokularlinse (32; 132; 232) dafür gestaltet ist, die von der primären Intraokularlinse (30) geleistete Sehkorrektur zu verstärken.

3. Intraokularlinsensystem gemäß einem der vorstehenden Ansprüche, wobei die Ergänzungs-Intraokularlinse (32; 132; 232) eine elastisch biegsame Linse umfasst.

4. Intraokularlinsensystem gemäß einem der vorstehenden Ansprüche, wobei die Ergänzungs-Intraokularlinse (32; 132; 232) eine Dicke von weniger als etwa 700 µm aufweist.

5. Intraokularlinsensystem gemäß einem der vorstehenden Ansprüche, wobei die Ergänzungs-Intraokularlinse (32; 132; 232) eine Dicke in dem Bereich von etwa 10 µm bis etwa 300 µm aufweist.

6. Intraokularlinsensystem gemäß Anspruch 5, wobei die Ergänzungs-Intraokularlinse (32; 132; 232) eine Dicke von nicht mehr als etwa 250 µm aufweist.

7. Intraokularlinsensystem gemäß einem der vorstehenden Ansprüche, wobei die Ergänzungs-Intraokularlinse (32; 132; 232) funktionsfähig an die primäre Intraokularlinse (30) gekoppelt ist.

8. Intraokularlinsensystem gemäß einem der vorstehenden Ansprüche, wobei die Ergänzungs-Intraokularlinse (32; 132; 232) eine positive optische Stärke aufweist.

9. Intraokularlinsensystem gemäß einem der vorstehenden Ansprüche, wobei die Ergänzungs-Intraokularlinse (32; 132; 232) eine negative optische Stärke aufweist.

10. Intraokularlinsensystem gemäß einem der vorstehenden Ansprüche, wobei die Ergänzungs-Intraokularlinse (32; 132; 232) getönt ist.

11. Intraokularlinsensystem gemäß Anspruch 10, wobei die Ergänzungs-Intraokularlinse (32; 132; 232) einen Blaublocker umfasst.

12. Intraokularlinsensystem gemäß einem der vorstehenden Ansprüche, wobei die Ergänzungs-Intraokularlinse (32; 132; 232) multifokal ist.

13. Intraokularlinsensystem gemäß einem der vorstehenden Ansprüche, wobei die Ergänzungs-Intraokularlinse (32; 132; 232) eine Brechkraft und eine Dicke aufweist, wobei die Brechkraft von der Dicke unabhängig ist.

14. Intraokularlinsensystem gemäß einem der vorstehenden Ansprüche, wobei die Ergänzungs-Intraokularlinse (32; 132; 232) eine Brechkraft aufweist und aus einem Material besteht, das einen Brechungsindex aufweist, wobei die Brechkraft der Ergänzungs-Intraokularlinse (32; 132; 232) von dem Brechungsindex des Materials unabhängig ist.

15. Intraokularlinsensystem gemäß einem der vorstehenden Ansprüche, wobei die primäre Intraokularlinse (30) Befestigungselemente (36, 38) zum Befestigen der primären Intraokularlinse in einem Auge eines Patienten aufweist und
die Ergänzungs-Intraokularlinse (32; 132; 232) zum Befestigen an der primären Intraokularlinse (30) gestaltet ist oder zur Implantation in das Auge des Patienten getrennt von der primären Intraokularlinse (30).

## Revendications

1. Système de lentille intraoculaire destiné à être inséré dans un oeil, comprenant :
une lentille intraoculaire primaire (30) configurée pour être placée dans l'oeil d'un patient ; et
une lentille intraoculaire supplémentaire (32 ; 132 ; 232) configurée pour être placée dans l'oeil du patient, la lentille intraoculaire supplémentaire (32 ; 132 ; 232) comprenant une optique pratiquement intégralement diffractive, **caractérisé en ce que** :
la lentille intraoculaire primaire (30) est configurée pour être efficace pour corriger la vision du patient et la lentille intraoculaire supplémentaire (32 ; 132 ; 232) est configurée pour modifier la correction de la vision procurée par la lentille intraoculaire primaire (30), et la lentille intraoculaire supplémentaire (32 ; 132 ; 232) est soit voûtée au niveau antérieur par rapport à la lentille intraoculaire primaire (30), soit torique.

2. Système de lentille intraoculaire selon la revendication 1, dans lequel la lentille intraoculaire supplémentaire (32 ; 132 ; 232) est configurée pour améliorer la correction de la vision procurée par la lentille intraoculaire primaire (30).

3. Système de lentille intraoculaire selon l'une quelconque des revendications précédentes, dans lequel la lentille intraoculaire supplémentaire (32 ; 132 ; 232) comprend une lentille pliable résiliente.

4. Système de lentille intraoculaire selon l'une quelconque des revendications précédentes, dans lequel la lentille intraoculaire supplémentaire (32 ; 132 ; 232) a une épaisseur de moins de 700 µm environ.

5. Système de lentille intraoculaire selon l'une quelconque des revendications précédentes, dans lequel la lentille intraoculaire supplémentaire (32 ; 132 ; 232) a une épaisseur comprise dans une plage s'étendant d'environ 10 µm à environ 300 µm.

6. Système de lentille intraoculaire selon la revendication 5, dans lequel la lentille intraoculaire supplémentaire (32 ; 132 ; 232) a une épaisseur n'excédant pas environ 250 µm.

7. Système de lentille intraoculaire selon l'une quelconque des revendications précédentes, dans lequel la lentille intraoculaire supplémentaire (32 ; 132 ; 232) est couplée de manière opérationnelle à la lentille intraoculaire primaire (30).

8. Système de lentille intraoculaire selon l'une quelconque des revendications précédentes, dans lequel la lentille intraoculaire supplémentaire (32 ; 132 ; 232) a une puissance optique positive.

9. Système de lentille intraoculaire selon l'une quelconque des revendications précédentes, dans lequel la lentille intraoculaire supplémentaire (32 ; 132 ; 232) a une puissance optique négative.

10. Système de lentille intraoculaire selon l'une quelconque des revendications précédentes, dans lequel la lentille intraoculaire supplémentaire (32 ; 132 ; 232) est tintée.

11. Système de lentille intraoculaire selon la revendication 10, dans lequel la lentille intraoculaire supplémentaire (32 ; 132 ; 232) comprend un dispositif arrêtant le rayonnement bleu.

12. Système de lentille intraoculaire selon l'une quelconque des revendications précédentes, dans lequel la lentille intraoculaire supplémentaire (32 ; 132 ; 232) est multifocale.

13. Système de lentille intraoculaire selon l'une quelconque des revendications précédentes, dans lequel la lentille intraoculaire supplémentaire (32 ; 132 ; 232) a une puissance de réfraction et une épaisseur, la puissance de réfraction étant indépendante de l'épaisseur.

14. Système de lentille intraoculaire selon l'une quelconque des revendications précédentes, dans lequel la lentille intraoculaire supplémentaire (32 ; 132 ; 232) a une puissance de réfraction et est formée d'un matériau ayant un indice de réfraction, la puissance de réfraction de la lentille intraoculaire supplémentaire (32 ; 132 ; 232) étant indépendante de l'indice de réfraction du matériau.

15. Système de lentille intraoculaire selon l'une quelconque des revendications précédentes, dans lequel la lentille intraoculaire primaire (30) comprend des éléments de fixation (36, 38) pour fixer la lentille intraoculaire primaire (30) dans l'oeil d'un patient, et la lentille intraoculaire supplémentaire (32 ; 132 ; 232) est configurée pour être fixée à la lentille intraoculaire primaire (30) ou pour être implantée dans l'oeil du patient de manière séparée par rapport à la lentille intraoculaire primaire (30).
